# EUROPÄISCHE PATENTSCHRIFT

(11) **EP 0 598 291 B1**
(45) Veröffentlichungstag und Bekanntmachung des Hinweises auf die Patenterteilung: **02.01.1997**
(21) Anmeldenummer: 93117953.5
(22) Anmeldetag: 05.11.1993
(51) Int. Cl.: A61F 13/08, A61F 13/06, A61F 13/10, A61L 15/30

(54) **Gelenkbandage**
Joint bandage
Bandage pour articulations

(30) Priorität: 16.11.1992 DE 4238610
(43) Veröffentlichungstag der Anmeldung: 25.05.1994
(73) Patentinhaber: FERD. HAUBER GmbH & CO. KG, D-72622 Nürtingen (DE)
(72) Erfinder: Berkowitsch, Ewald, D-73230 Kirchheim (DE)
(74) Vertreter: Becker, Maria, Dipl.-Phys.

(56) Entgegenhaltungen:
- EP-A- 0 115 029
- DE-A- 2 936 174
- DE-C- 3 412 772

## Beschreibung

Die Erfindung betrifft eine Gelenkbandage, wie sie im Oberbegriff des Patentanspruchs 1 angegeben ist.

Bei einer derartigen bekannten Gelenkbandage (DE 29 36 174 A1) ist in einem strumpfförmigen Schlauch aus elastischem Bandagenstoff, der für die Abstützung bzw. Kompression von Knie-, Sprung-, Ellenbogen- und/oder Handgelenken dient, eine Kompressionseinlage vorgesehen, die in Gelenkweichteile, also in konkave Räume des Gelenks, drückbar ist.

Bei einer bekannten Kniebandage oder Orthese (DE 34 12 772 C1) ist im Bereich der Kniescheibe ein ringförmiges Druckpolster oder Pelotte an einem strumpfförmigen Schlauch aus dehnbarem Gestrick angeordnet. Das die Kniescheibe bei angelegter Kniebandage umgebende ringförmige Druckpolster weist dabei auf seiner dem Gelenk zugewandten Seite eine wulstartige Erhöhung aus weichem, druckfestem Werkstoff auf, die zur wirkungsvolleran Medialisierung der Kniescheibe dient.

Ausgehend von diesem Stand der Technik liegt der Erfindung die Aufgabe zugrunde, eine weitere Gelenkbandage der eingangs genannten Art bereitzustellen; insbesondere soll diese eine verbesserte Massagewirkung auf das jeweilige Gelenk ausüben.

Erfindungsgemäss wird diese Aufgabe bei einer Gelenkbandage der eingangs genannten Art durch die kennzeichnenden Merkmale des Patentanspruchs 1 gelöst.

Erfindungsgemäss wird also die dem Gelenk zugewandte Oberfläche mit noppenartigen Vorsprüngen versehen, um die Reibung zwischen dem Druckpolster und der Haut des Patienten im Bereich des bandagierten Gelenks zu erhöhen. Hierdurch wird erreicht, dass Relativbewegungen zwischen dem Druckpolster und dem Gelenk, die bei der Bewegung des Gelenkes auftreten, zu einer Massage des Gelenks, insbesondere der Gelenkweichteile, führt.

Erste praktische Ausgestaltungen der Erfindung sind in den Ansprüchen 2 bis 5 beschrieben. Obwohl es möglich ist, die noppenartigen Vorsprünge in einem beliebigen Muster anzuordnen, ist die rasterförmige Anordnung vorzuziehen, da sich auf diese Weise die Reibung zwischen dem Druckpolster und der Haut des Patienten besonders gut vorgeben lässt.

Eine besonders vorteilhafte Weiterbildung der Erfindung ist in Anspruch 6 beschrieben, wobei vorzugsweise entsprechend Anspruch 7 die die Vorsprünge aufweisende Schicht aus einem festeren Material besteht als die dem strumpfförmigen Schlauch benachbarte Schicht des Druckpolsters. Hierdurch wird erreicht, dass das Druckpolster der erfindungsgemässen Bandage elastisch genug ist, um vom strumpfförmigen Schlauch auch bei Bewegungen gut an das Gelenk angedrückt wird. Gleichzeitig sind die Vorsprünge und die diese tragende Schicht fest genug, um die erwünschte Massagewirkung zu erzielen.

Zweckmässige Weiterbildungen der Erfindung sind in den Ansprüchen 8 und 9 beschrieben.

Um eine unerwünschte Druckbelastung bestimmter Galenkabschnitte zu vermeiden, ist die Ausgestaltung nach Anspruch 10 vorgesehen. Eine derartige radiale rinnenförmige Ausnehmung kann beispielsweise bei einer Gelenkbandage, die für das Kniegelenk vorgesehen ist, so angeordnet werden, dass sie der unterhalb der Kniescheibe verlaufenden Sehne zugeordnet ist und somit einen Druck auf diese verhindert.

Dabei kann sich die Ausnehmung über die gesamte radiale Breite oder - wie bei den bevorzugten Ausgestaltungen nach Anspruch 11 und 12 - nur über einen radial aussenliegenden Teil davon erstrecken.

Dabei bewirkt das Druckpolster bei einer für ein Knie ausgebildeten Gelenkbandage eine Hyperämisierung des parapatellaren Bereiches und damit eine Durchblutungsverbesserung auch des Knieinnenraumes. Damit ergibt sich eine verbesserte Ernährungssituation für den Knorpel und eine beschleunigtere Resorption von Ergüssen. Durch die vom Druckpolster ausgeübte zirkuläre Reibungsmassage der patellaren Insertionen lässt sich eine günstige therapeutische Beeinflussung von Ligamentosen und Insertionstendopathien im Bereich der Patella erreichen.

Der mit noppenartigen Vorsprüngen versehene Steg, der bei korrekt angelegter Bandage am Ursprung des Ligamentum patellae anliegt und an den sich distal, also bezogen auf das ringförmige Druckpolster radial aussenliegend, die Ausnehmung mit glatter Oberfläche anschliesst, bewirkt eine Reibungsmassage auch an der Patellaspitze (Patellaspitzensyndrom) bei Vermeidung eventuell unangenehmer Irritation des Ligamentum patellae und der hochempfindlichen kranialen Begrenzung der Tuberositas tibiae durch die glatte Ausnehmung.

Eine besonders hautschonende Gelenkbandage ist in Anspruch 13 beschrieben. Das dünne Textilmaterial dient dabei nicht nur zur Hautschonung und zur Vermittlung eines angenehmen Tragegefühls bei angelegter Bandage sondern auch zur Anpassung der Reibwirkung. Insbesondere lässt sich durch eine entsprechende Abstimmung der Dicke des Textilmaterials auf die Grösse der Vorsprünge die jeweils gewünschte Reib- und Massagewirkung erreichen.

Um eine Relativbewegung zwischen dem Druckpolster und dem strumpfförmigen Schlauch der Gelenkbandage zu ermöglichen, sind die Ausgestaltungen nach Anspruch 14 und 15 vorgesehen.

Hierdurch wird die Stützwirkung und der Tragekomfort der Gelenkbandage verbessert und gleichzeitig eine Abschnürung des Gelenks verhindert, da der strumpfförmige Schlauch der Gelenkbewegung ungehindert folgen kann, während das Druckpolster im wesentlichen in seiner Lage gehalten wird.

Die Erfindung wird im folgenden beispielsweise anhand der Zeichnung näher erläutert. Es zeigen:
- Fig. 1: eine Draufsicht auf eine Gelenkbandage, wobei einzelne Materialschichten teilweise weggebrochen sind;
- Fig. 2: eine Draufsicht auf ein Druckpolster für die Gelenkbandage nach Fig. 1;
- Fig. 3: einen Schnitt im wesentlichen nach Linie III-III in Fig. 2;
- Fig. 4: einen Schnitt im wesentlichen nach Linie IV-IV in Fig. 2;
- Fig. 5: eine Teildraufsicht auf ein anderes Druckpolster ähnlich Fig. 2;
- Fig. 6: einen Schnitt im wesentlichen nach Linie VI-VI in Fig. 5.

In den verschiedenen Figuren der Zeichnung sind einander entsprechende Teile mit gleichen Bezugszeichen versehen.

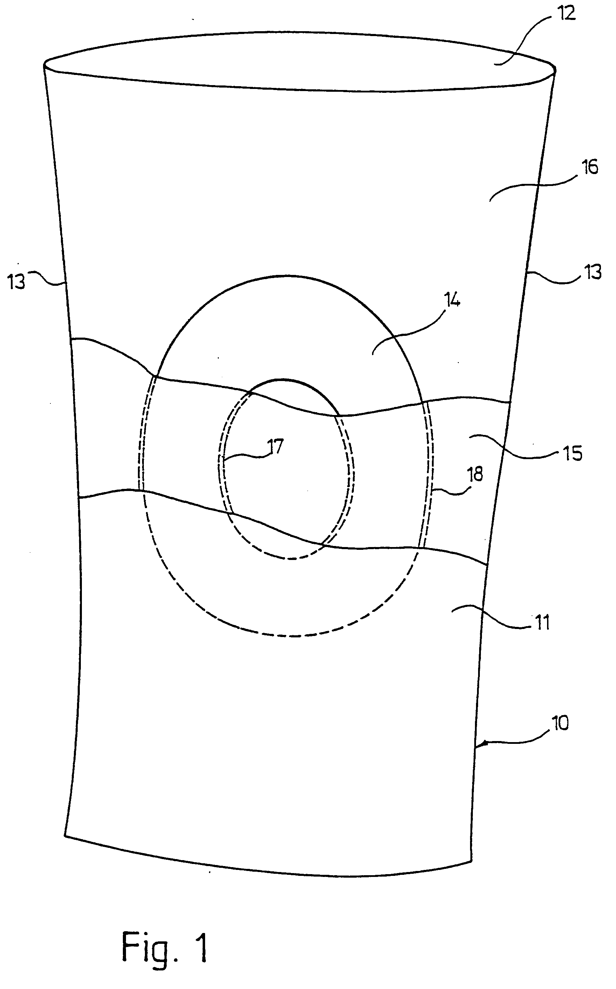

Die in Fig. 1 gezeigte Gelenkbandage, die als Kniebandage ausgebildet ist, weist einen strumpfförmigen Schlauch 10 auf, der aus einer vorderen und einer hinteren Bandagenstofflage 11 bzw. 12 besteht. Die beiden Bandagenstofflagen 11, 12 sind dabei mit ihren Seitenlängskanten 13 miteinander verbunden.

Wird als Bandagenstoff ein elastisches, dehnbares Gestrick verwendet, so erfolgt die Verbindung der beiden Bandagenstofflagen 11, 12 vorzugsweise durch Nähen.

Innerhalb des strumpfförmigen Schlauches 10 der Kniebandage ist ein ringförmiges Druckpolster 14 angeordnet, das bei sachgerecht angelegter Kniebandage die Kniescheibe so umgibt, dass es in die die Kniescheibe umgebenden konkaven Räume drückt. Das Druckpolster 14 ist dabei zwischen zwei Stofflagen 15, 16 angeordnet, die etwa die gleiche Form aufweisen wie die vordere Bandagenstofflage 11 und die miteinander und mit dieser im Bereich der Seitenlängskanten 13 vernäht sind. Um das ringförmige Druckpolster 14 in der Kniebandage so zu fixieren, dass es stets in seiner korrekten Position gehalten ist, sind die beiden Stofflagen 15, 16 durch ringförmige Nähte 17, 18 miteinander verbunden, so dass sich das Druckpolster 14 gegenüber den Stofflagen 15, 16 nicht verschieben kann.

Für die das Druckpolster haltenden Stofflagen 15, 16 wird vorzugsweise ein dünnes elastisches Textilmaterial verwendet, so dass sich das Druckpolster 14 gegenüber der vorderen Bandagenstofflage 11 geringfügig verschieben lässt.

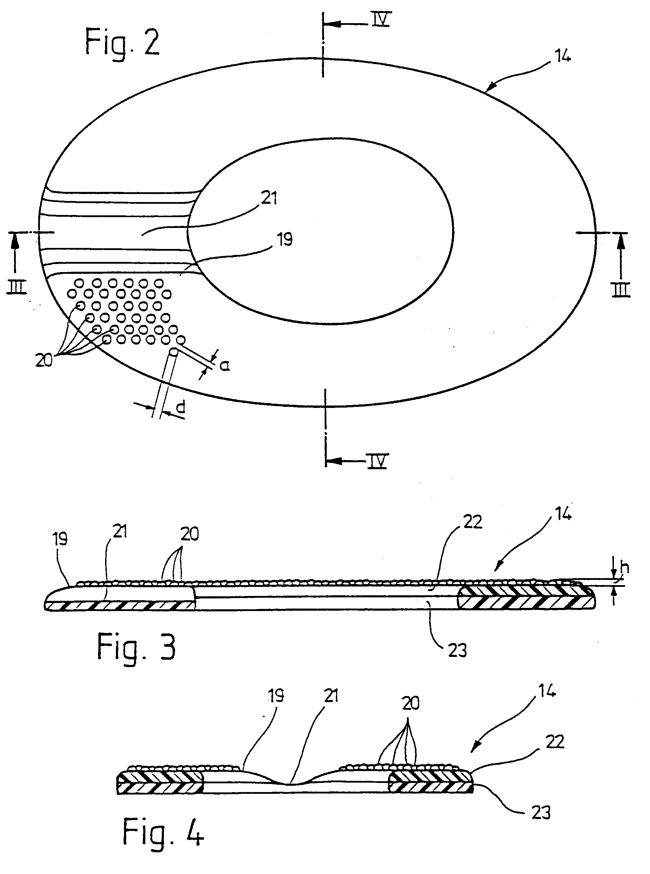

Entsprechend den Fig. 2 bis 4 weist das ringförmige Druckpolster 14 auf seiner in der Bandage nach innen gerichteten Oberfläche 19 eine Vielzahl von noppenartigen Vorsprüngen oder Noppen 20 auf, die, wie Fig. 2 zeigt vorteilhafterweise in einem regelmässigen Muster so angeordnet sind, dass - abgesehen von den am Rand befindlichen Noppen jede Noppe 20 von sechs unmittelbar benachbarten Noppen 20 umgeben ist, die den gleichen Abstand von der mittleren Noppe 20 aufweisen. Die Höhe h der Noppen 20 liegt zwischen 0,5 mm und 2 mm und beträgt vorzugsweise 1 mm, während der Durchmesser d der Noppen 20 im Bereich der Oberfläche 19, der zwischen 1,5 mm und 6 mm liegen kann, vorzugsweise 3 mm beträgt. Der Abstand a der Noppen im Bereich der Oberfläche 19 entspricht dabei im wesentlichen ihrer Höhe h und ist im dargestellten Ausführungsbeispiel 1 mm.

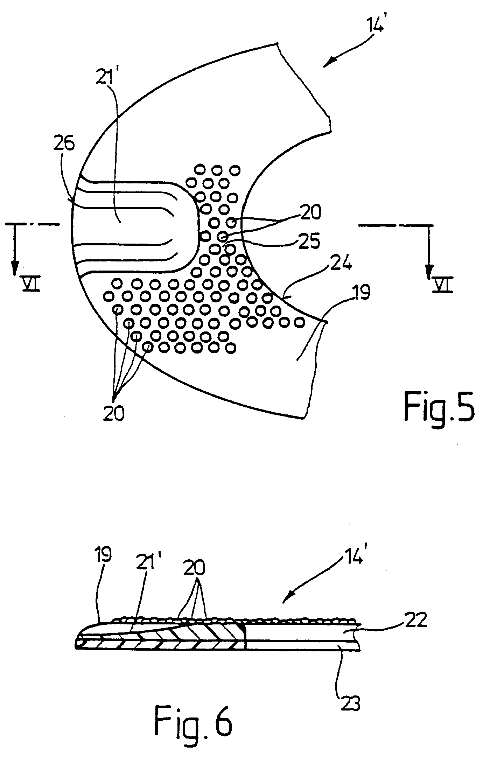

Das Druckpolster 14 weist ferner eine zur Oberfläche 19 hin offene, sich radial erstreckende Rille 21 auf, die, wie Fig. 4 zeigt, einen flachen, V-förmigen Querschnitt besitzt. Diese Rille 21 ist so am ringförmigen Druckpolster 14 angeordnet, dass sie bei richtig angelegter Kniebandage unterhalb der Kniescheibe zu liegen kommt und die dort verlaufende Sahne überdeckt, so dass auf diese kein Druck ausgeübt wird.

Um das ringförmige Druckpolster 14 so elastisch auszubilden, dass es einerseits weich genug ist, um mit seiner noppenbesetzten Oberfläche 19 von dem Bandagenstoff in engem Kontakt an dem Gelenk gehalten zu werden, und andererseits fest genug ist, um mit seinen Noppen 20 eine Massagewirkung auf die Gelenkweichteile, z.B. auf die Gewebebereiche neben der Kniescheibe, ausüben zu können, ist das Druckpolster 14 aus zwei Schichten 22, 23 aufgebaut. Die in den Fig. 3 und 4 untere Schicht 23 besteht dabei aus einem weichen Material, während die obere, dem Gelenk zugewandte und die Noppen 20 tragende Schicht 22 aus einem relativ festen Material besteht. Vorzugsweise wird für beide Schichten 22, 23 Silikonkautschuk verwendet, so dass die beiden Schichten 22, 23 dauerhaft miteinander verbunden werden können.

Bei sachgerecht am Knie angelegter Kniebandage liegt das Druckpolster 14 mit seinen Noppen 20 über die vorzugsweise dünne, textile Stofflage 16 auf dem Kniegelenk auf, wobei die Kniescheibe und die unterhalb der Kniescheibe verlaufende Sehne nicht beaufschlagt werden. Die Noppen 20 bewirken dabei eine Reibung zwischen dem Druckpolster 14 und der Haut, wodurch bei der Bewegung des Knies eine Massagewirkung erzielt wird. Hierdurch wird eine verbesserte Durchblutung des entsprechenden Gelenks erreicht, wodurch der Abbau von Blutergüssen und die Heilung von Verletzungen beschleunigt wird.

In Fig. 5 und 6 ist ein anderes ringförmiges Druckpolster 14' dargestellt, bei dem eine zur Oberfläche 19 hin offene, sich radial erstreckende Rille 21' vorgesehen ist, die mit Abstand vom radial innenliegenden Rand 24 des ringförmigen Druckpolsters 14' unter Bildung eines Steges 25 beginnt und sich bis zum radial äusseren Rand 26 hin erstreckt. Der zwischen der Rille 21' und dem inneren Rand 24 gebildete Steg 25 weist auf seiner Oberfläche, wie die Oberfläche 19 des übrigen Druckpolsters auch, noppenartige Vorsprünge 20 auf.

Bei einer korrekt angelegten Kniebandage mit dem Druckpolster nach Fig. 5 und 6 liegt der Steg 25 mit seinen noppenartigen Vorsprüngen an der Patellaspitze, also der Kniescheibenspitze, an und mitbewirkt dort eine Massage, was insbesondere bei einem Patellaspitzensyndrom von Vorteil ist. Die distal angrenzende Ausnehmung 21 mit glatter Oberfläche vermeidet dabei eventuell unangenehme Irritationen des Ligamentum patellae und der hoch empfindlichen kranialen Begrenzung der Tuberositas tibiae.

## Patentansprüche

1. Gelenkbandage
- mit einem strumpfförmigen Schlauch (10) aus elastischem, dehnbarem Material und
- mit einem elastischen Druckpolster (14), das auf der Innenseite des Schlauches (10) an diesem gehalten ist und das bei angelegter Bandage in konkave Räume des jeweiligen Gelenks drückbar ist,
**dadurch gekennzeichnet,**
- dass das Druckpolster (14) auf seiner im wesentlichen ganzen, dem Gelenk zugewandten Oberfläche (19) mit noppenartigen Vorsprüngen (20) versehen ist.

2. Gelenkbandage nach Anspruch 1, dadurch gekennzeichnet, dass die noppenartigen Vorsprünge 0,5 mm bis 2 mm, insbesondere 0,7 mm bis 1,5 mm, vorzugsweise 1 mm, über die dem Gelenk zugewandte Oberfläche (19) vorstehen.

3. Gelenkbandage nach Anspruch 1 oder 2, dadurch gekennzeichnet, dass die noppenartigen Vorsprünge (20) kugelabschnittförmig ausgebildet sind und im Bereich der dem Gelenk Zugewandten Oberfläche (19) einen Durchmesser (d) von 1,5 mm bis 6 mm, insbesondere von 2 mm bis 7 mm, vorzugszweise von 3 mm, aufweisen.

4. Gelenkbandage nach Anspruch 1, 2 oder 3, dadurch gekennzeichnet, dass die noppenartigen Vorsprünge (20) einen Abstand (a) von 0,5 mm bis 2 mm, insbesondere von 0,7 mm bis 1,5 mm, vorzugsweise von 1 mm, voneinander aufweisen.

5. Gelenkbandage nach einem der vorhergehenden Ansprüche, dadurch gekennzeichnet, dass die noppenartigen Vorsprünge (20) in einem regelmässigen Raster angeordnet sind, wobei jeder im Innenbereich des Rastars angeordnete Vorsprung von jeweils sechs unmittelbar benachbarten Vorsprüngen den gleichen Abstand aufweist.

6. Gelenkbandage nach einem der vorhergehenden Ansprüche, dadurch gekennzeichnet, dass das Druckpolster (14) zwei Materialschichten (22, 23) aufweist.

7. Gelenkbandage nach Anspruch 6, dadurch gekennzeichnet, dass die die Vorsprünge (20) aufweisende Schicht (22) aus einem festeren Material besteht als die dem strumpfförmigen Schlauch (10) benachbarte Schicht des Druckpolsters (14).

8. Gelenkbandage nach Anspruch 6 oder 7, dadurch gekennzeichnet, dass die beiden Schichten (22, 23) des Druckpolsters mit ihren aneinanderliegenden Flächen fest miteinander verbunden sind.

9. Gelenkbandage nach einem der vorhergehenden Ansprüche, dadurch gekennzeichnet, dass das Druckpolster (14) aus Silikonkautschuk besteht.

10. Gelenkbandage nach einem dar vorhergehenden Ansprüche, dadurch gekennzeichnet, dass das Druckpolster (14) ringförmig ausgebildet ist und an seiner dem Gelenk zugewandten Seite eine radiale, rillenförmige Ausnehmung (21) aufweist.

11. Gelenkbandage nach Anspruch 10, dadurch gekennzeichnet, dass die radiale Ausnehmung (21) mit Abstand zum radial innenliegenden Rand (24) des ringförmigen Druckpolsters (14) endet, so dass ein radial innenliegender, sich umfangsmässig erstreckender Steg (25) gebildet ist.

12. Gelenkbandage nach Anspruch 11, dadurch gekennzeichnet, dass die dem Gelenk zugewandte Oberfläche des Steges (25) mit noppenartigen Vorsprüngen (20) versehen ist, während die Ausnehmung (20) eine glatte Oberfläche aufweist.

13. Gelenkbandage nach einem der vorhergehenden Ansprüche, dadurch gekennzeichnet, dass das Druckpolster (14) auf seiner dem Gelenk zugewandten Seite von einem dünnen Textilmaterial (16) überdeckt ist.

14. Gelenkbandage nach Anspruch 13, dadurch gekennzeichnet, dass das Druckpolster in einer Tasche angeordnet ist, die innen im strumpfförmigen Schlauch (10) vorgesehen und an diesem befestigt ist.

15. Gelenkbandage nach Anspruch 13 oder 14, dadurch gekennzeichnet, dass die Tasche für das Druckpolster (14) an einer elastischen Textilschicht (15, 16) vorgesehen ist, die mit Abstand zur Aussenkontur des Druckpolsters (14) innen an dem strumpfförmigen Schlauch (10) befestigt ist.

## Claims

1. Joint bandage having
- a hose-like tubing (10) consisting of an elastic, extensible material and
- an elastic pressure pad (14), which is retained on the inside of the tubing and can be pressed into concave spaces of the respective joint when the bandage is in place,
characterised in that
- the pressure pad (14) is provided with knob-like projections (20) on substantially its entire surface (19) facing the joint.

2. Joint bandage according to claim 1, characterised in that the knob-like projections project by 0.5 mm to 2 mm, especially by 0.7 mm to 1.5 mm, preferably by 1 mm, over the surface (19) facing the joint.

3. Joint bandage according to claim 1 or 2, characterised in that the knob-like projections (20) have the shape of spherical segments and a diameter (d), in the area of the surface (19) facing the joint, of 1.5 mm to 6 mm, especially 2 mm to 7 mm, preferably 3 mm.

4. Joint bandage according to claim 1, 2 or 3, characterised in that the knob-like projections (20) are spaced one from the other by a distance (a) of 0.5 mm to 2 mm, especially 0.7 mm to 1.5 mm, preferably 1 mm.

5. Joint bandage according to any of the preceding claims, characterised in that the knob-like projections (20) are arranged in a regular grid pattern, with each projection located in an inner area of the grid being spaced from six directly neighbouring projections by the same distance.

6. Joint bandage according to any of the preceding claims, characterised in that the pressure pad (14) comprises two layers of material (22, 23).

7. Joint bandage according to claim 6, characterised in that the layer (22) comprising the projections (20) is made from a stronger material than the layer of the pressure pad (14) adjacent the hose-like tubing (10).

8. Joint bandage according to claim 6 or 7, characterised in that the two layers (22, 23) of the pressure pad are firmly connected by their respective adjacent surfaces.

9. Joint bandage according to any of the preceding claims, characterised in that the pressure pad (14) consists of silicon caoutchouc.

10. Joint bandage according to any of the preceding claims, characterised in that the pressure pad (14) exhibits an annular shape and comprises a radial, groove-like recess (21) on its side facing the joint.

11. Joint bandage according to claim 10, characterised in that the radial recess (21) terminates at a distance from a radially inner edge (24) of the annular pressure pad (14) so as to provide a radially inner, circumferentially extending rib (25).

12. Joint bandage according to claim 11, characterised in that the surface of the rib (25) facing the joint is provided with knob-like projections (20), whereas the recess (21) has a smooth surface.

13. Joint bandage according to any of the preceding claims, characterised in that the pressure pad (14) is covered by a thin textile material (16) on its side facing the joint.

14. Joint bandage according to claim 13, characterised in that the pressure pad is arranged in a pocket, which is provided inside the hose-like tubing (10) and is fixed to the latter.

15. Joint bandage according to claim 13 or 14, characterised in that the pocket for the pressure pad (14) is arranged on an elastic textile layer (15, 16) and is fixed on the inside of the hose-like tubing (10) at a distance from the outer contour of the pressure pad (14).

## Revendications

1. Bandage pour articulations
- avec un boyau (10) en forme de bas en un matériau élastique, extensible et
- avec un rembourrage compressif (14) élastique maintenu sur la face intérieure du boyau (10) et pouvant être pressé dans les espaces concaves de l'articulation respective une fois le bandage appliqué,
**caractérisé en ce que**
- le rembourrage compressif (10) est muni sur presque toute sa surface (19) tournée vers l'articulation de saillies en forme de nope (20).

2. Bandage pour articulations selon la revendication 1, caractérisé en ce que les saillies en forme de nope dépassent la surface (19) tournée vers l'articulation de 0,5 mm à 2 mm, essentiellement de 0,7 mm à 1,5 mm, de préférence de 1 mm.

3. Bandage pour articulations selon la revendication 1 ou 2, caractérisé en ce que les salles en forme de nope (20) ont une configuration en forme de segment sphérique et présentent dans la zone de la surface (19) tournée vers l'articulation un diamètre (d) de 1,5 mm à 6 mm, essentiellement de 2 mm à 7 mm, de préférence de 3 mm.

4. Bandage pour articulations selon la revendication 1, 2 ou 3, caractérisé en ce que les saillies en forme de nope (20) présentent un écart (a) entre elles de 0,5 mm à 2 mm, essentiellement de 0,7 mm à 1,5 mm de préférence de 1 mm.

5. Bandage pour articulations selon l'une des revendications précédentes, caractérisé en ce que les saillies en forme de nope (20) sont disposées en une trame régulière, chaque saillie disposée dans la zone intérieure de la trame présentant le même écart de respectivement six saillies directement contiguës.

6. Bandage pour articulations selon l'une des revendications précédentes, caractérisé en ce que le rembourrage compressif (14) présente deux couches de matériaux (22, 23).

7. Bandage pour articulations selon la revendication 6, caractérisé en ce que la couche (22) présentant les saillies (20) consiste en un matériau plus solide que la couche du rembourrage compressif (14) contiguë au boyau (10) en forme de bas.

8. Bandage pour articulations selon la revendication 6 ou 7, caractérisé en ce que les deux couches (22, 23) de rembourrage compressif sont reliées fermement par leurs surfaces attenantes.

9. Bandage pour articulations selon l'une des revendications précédentes, caractérisé en ce que le rembourrage compressif (14) est en caoutchouc au silicone.

10. Bandage pour articulations selon l'une des revendications précédentes, caractérisé en ce que le rembourrage compressif (14) a une configuration annulaire et présente sur sa face tournée vers l'articulation un évidement radial (21) en forme de rainure.

11. Bandage pour articulations selon la revendication 10, caractérisé en ce que l'évidement radial (21) se termine avec un écart du bord radial (24) intérieur du rembourrage compressif (14) annulaire, de sorte à former une nervure radiale intérieure (25) s'étendant circonférentiellement.

12. Bandage pour articulations selon a revendication 11, caractérisé en ce que la surface de la nervure (25) tournée vers l'articulation est munie de saillies (20) en forme de nope, alors que l'évidement (21) présente une surface lisse.

13. Bandage pour articulations selon l'une des revendications précédentes, caractérisé en ce que le rembourrage compressif (14) est recouvert d'un mince matériau textil (16) sur sa face tournée vers l'articulation.

14. Bandage pour articulations selon la revendication 13, caractérisé en ce que le rembourrage compressif est disposé dans une poche prévue à l'intérieur du boyau (10) en forme de bas et fixée sur ce dernier.

15. Bandage pour articulations selon la revendication 13 ou 14, caractérisé en ce que la poche pour le rembourrage compressif (14) est prévue sur une couche textile élastique (15, 16) qui est fixée sur le boyau (10) en forme de bas avec un écart du contour extérieur du rembourrage compressif (14).
